# EUROPÄISCHE PATENTSCHRIFT

(11) **EP 1 076 651 B1**
(45) Veröffentlichungstag und Bekanntmachung des Hinweises auf die Patenterteilung: **20.04.2005**
(21) Anmeldenummer: 99948554.3
(22) Anmeldetag: 30.04.1999
(51) Int. Cl.: C07D 233/90, C07D 233/68, A61K 31/415

(54) **SUBSTITUIERTE SULFONYLCYANAMIDE, VERFAHREN ZU IHRER HERSTELLUNG UND IHRE VERWENDUNG ALS MEDIKAMENT**
SUBSTITUTED SULPHONYL CYANAMIDES, METHOD FOR PRODUCING SAME AND THEIR USE AS MEDICAMENT
SULFONYLCYANAMIDES SUBSTITUES, LEUR PROCEDE DE PRODUCTION ET LEUR UTILISATION COMME MEDICAMENT

(30) Priorität: 06.05.1998 DE 19820064
(43) Veröffentlichungstag der Anmeldung: 21.02.2001
(73) Patentinhaber: Aventis Pharma Deutschland GmbH, 65929 Frankfurt am Main (DE)
(72) Erfinder: WEICHERT, Andreas, D-63329 Egelsbach (DE); LANG, Hans, Jochen, D-65719 Hofheim (DE); PETRY, Stefan, D-65929 Frankfurt am Main (DE); SCHWARK, Jan-Robert, D-65779 Kelkheim (DE); KLEEMANN, Heinz-Werner, D-65474 Bischofsheim (DE); FABER, Sabine, D-65510 Idstein (DE); JANSEN, Hans-Willi, D-65527 Niedernhausen (DE)
(86) Internationale Anmeldenummer: PCT/EP1999/002940
(87) Internationale Veröffentlichungsnummer: WO 1999/057102

(56) Entgegenhaltungen:
- EP-A- 0 253 310
- EP-A- 0 465 368
- EP-A- 0 479 479
- EP-A- 0 855 392
- EP-A- 0 903 339
- WO-A-95/23791
- US-A- 5 482 957
- US-A- 5 604 251

## Beschreibung

Die Erfindung betrifft Verbindungen der Formel (I), 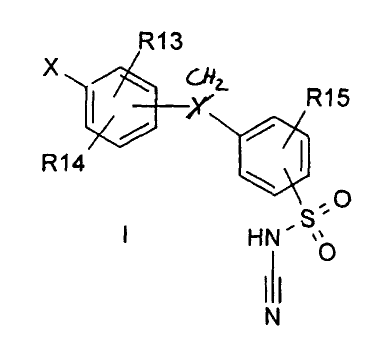 in welcher die Symbole folgende Bedeutung haben:
- X: 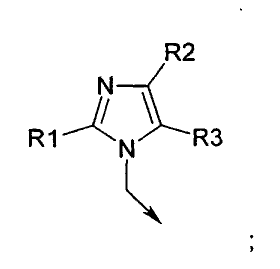
- R(1): Alkyl mit 1, 2, 3 oder 4 C-Atomen oder Phenyl, das unsubstituiert oder substituiert ist mit einem Rest aus der Reihe F, Cl, CF₃, Methyl oder Methoxy;
- R(2) und R(3): unabhängig voneinander
Wasserstoff, F, Cl, CF₃, -CN, CO-R(24) oder O-R(25),
R(24) Wasserstoff, Alkyl mit 1,2,3 oder 4 C-Atomen, OR(26) oder Phenyl, das unsubstituiert oder substituiert ist mit einem Rest aus der Reihe F, Cl, CF₃, Methyl oder Methoxy;
R(26) Wasserstoff, Methyl oder Ethyl;
R(25) Wasserstoff, Alkyl mit 1,2,3 oder 4 C-Atomen, Phenyl, das unsubstituiert oder substituiert ist mit einem Rest aus der Reihe F, Cl, CF₃, Methyl oder Methoxy;
oder
R(25) Heteroaryl, das unsubstituiert oder substituiert und mit einem Rest aus der Reihe F, Cl, CF₃, CH₃ oder Methoxy; oder
- R(2) und R(3): unabhängig voneinander
Alkyl mit 1, 2, 3 oder 4 C-Atomen, Cycloalkyl mit 3, 4, 5, 6 oder 7 C-Atomen;
oder
- R(2) und R(3): unabhängig voneinander
Phenyl, das unsubstituiert oder substituiert ist mit einem Rest aus der Reihe F, Cl, CF₃, Methyl oder Methoxy;
oder
- R(2) und R(3): unabhängig voneinander
Heteroaryl mit 1, 2, 3, 4, 5, 6, 7, 8 oder 9 C-Atomen, das unsubstituiert oder substituiert ist mit einem Rest aus der Reihe F, Cl, CF₃, CH₃ oder Methoxy;
oder
- R(2) und R(3): unabhängig voneinander
-SO_{f}-R(37),
R(37) Alkyl mit 1,2,3 oder 4 C-Atomen oder Phenyl, das unsubstituiert oder substituiert ist mit einem Rest aus der Reihe F, Cl, CF₃, Methyl oder Methoxy;
f Null oder 2;
- R(13), R(14) und R(15): unabhängig voneinander
Wasserstoff, Methyl, F, Cl, CF₃, -CN, SO₂-R(79), CO-R(80) oder O-R(81);
- R(79) und R(81): unabhängig voneinander
Alkyl mit 1, 2, 3 oder 4 C-Atomen oder Phenyl, das unsubstituiert ist oder substituiert ist mit einem Rest aus der Reihe F, Cl, CF₃, Methyl
oder Methoxy;
- R(80): Wasserstoff, Methyl oder OR(84);
R(84) Wasserstoff oder Alkyl mit 1, 2, 3, oder 4 C-Atomen;
sowie deren physiologisch verträgliche Salze.

Weiterhin sind Verbindungen der Formel I bevorzugt, in denen R(13), R(14), und R(15) jeweils Wasserstoff bedeutet, sowie deren physiologisch verträglichen Salze.

Alkyl kann geradkettig oder verzweigt sein.

Beipiele für Alkylreste mit 1, 2, 3, 4, 5, 6, 7 oder 8 C-Atomen sind: Methyl, Ethyl, Propyl, Butyl, Pentyl, Hexyl, Heptyl, Octyl, Isopropyl, Isobutyl, Isopentyl, Neopentyl, Isohexyl, 3-Methylpentyl, sec-Butyl, tert-Butyl, tert-Pentyl.

Unter Cycloalkyl werden auch Alkyl-substituierte Ringe verstanden.

Cycloalkylreste sind insbesondere Cyclopropyl, Cyclobutyl, Cyclopentyl, Cyclohexyl, Cycloheptyl, die aber auch beispiesweise durch Alkyl mit 1, 2, 3 oder 4 C-Atomen substituiert sein können. Als Beispiel für substituierte Cycloalkylreste seien 4-Methylcyclohexyl und 2,3-Dimethylcyclopentyl genannt.

Unter Heteroaryl mit 1, 2, 3, 4, 5, 6, 7, 8 oder 9 C-Atomen werden insbesondere Reste verstanden, die sich von Phenyl oder Naphthyl ableiten, in welchen eine oder mehrere CH-Gruppen durch N ersetzt sind und/oder in welchen mindestens zwei benachbarte CH-Gruppen (unter Bildung eines fünfgliedrigen aromatischen Rings) durch S, NH oder O ersetzt sind. Des weiteren können auch ein oder beide Atome der Kondensationsstelle bicyclischer Reste (wie im Indolizinyl) N-Atome sein.

Als Heteroaryl gelten insbesondere Furanyl, Thienyl, Pyrrolyl, Imidazolyl, Pyrazolyl, Triazolyl, Tetrazolyl, Oxazolyl, Isoxazolyl, Thiazolyl, Isothiazolyl, Pyridyl, Pyrazinyl, Pyrimidinyl, Pyridazinyl, Indolyl, Indazolyl, Chinolyl, Isochinolyl, Phthalazinyl, Chinoxalinyl, Chinazolinyl, Cinnolinyl.

Die vorliegende Erfindung umfaßt alle stereoisomeren Formen der Verbindungen der Formel I. In den Verbindungen der Formel I enthaltene Asymmetriezentren können alle unabhängig voneinander die S-Konfiguration oder die R-Konfiguration aufweisen. Zur Erfindung gehören alle möglichen Enantiomeren und Diastereomeren, ebenso Mischungen von zwei oder mehr stereoisomeren Formen, zum Beispiel Mischungen von Enantiomeren und/oder Diastereomeren, in allen Verhältnissen. Enantiomere sind also in enantiomerenreiner Form, sowohl als linksdrehende als auch als rechtsdrehende Antipoden, in Form von Racematen und in Form von Mischungen der beiden Enantiomeren in allen Verhältnissen Gegenstand der Erfindung. Bei Vorliegen einer cis/trans-Isomerie sind sowohl die cis-Form als auch die trans-Form und Gemische dieser Formen in allen Verhältnissen Gegenstand der Erfindung. Die Herstellung von einzelnen Stereoisomeren kann gewünschtenfalls durch Auftrennung eines Gemisches nach üblichen Methoden, zum Beispiel durch Chromatographie oder Kristallisation, durch Verwendung von stereochemisch einheitlichen Ausgangssubstanzen bei der Synthese oder durch stereoselektive Synthese erfolgen. Gegebenenfalls kann vor einer Trennung von Stereoisomeren eine Derivatisierung erfolgen. Die Trennung eines Stereoisomerengemisches kann auf der Stufe der Verbindungen der Formel I erfolgen oder auf der Stufe eines Zwischenprodukts im Verlaufe der Synthese. Bei Vorliegen von beweglichen Wasserstoffatomen umfaßt die vorliegende Erfindung auch alle tautomeren Formen der Verbindungen der Formel I.

Enthalten die Verbindungen der Formel I eine oder mehrere saure oder basische Gruppen, so sind auch die entsprechenden physiologisch oder toxikologisch verträglichen Salze Gegenstand der Erfindung, insbesondere die pharmazeutisch verwendbaren Salze. So können die Verbindungen der Formel I, die saure Gruppen enthalten, an diesen Gruppen beispielsweise als Alkalimetallsalze, Erdalkalimetallsalze oder als Ammoniumsalze vorliegen und erfindungsgemäß verwendet werden. Beispiele für solche Salze sind Natriumsalze, Kaliumsalze, Calciumsalze, Magnesiumsalze oder Salze mit Ammoniak oder organischen Aminen wie beispielsweise Ethylamin, Ethanolamin, Triethanolamin oder Aminosäuren. Verbindungen der Formel I, die eine oder mehrere basische, das heißt protonierbare, Gruppen enthalten, können in Form ihrer Säureadditionssalze mit physiologisch verträglichen anorganischen oder organischen Säuren vorliegen und erfindungsgemäß verwendet werden, zum Beispiel als Salze mit Chlorwasserstoff, Bromwasserstoff, Phosphorsäure, Schwefelsäure, Salpetersäure, Methansulfonsäure, p-Toluolsulfonsäure, Naphthalindisulfonsäuren, Oxalsäure, Essigsäure, Weinsäure, Milchsäure, Salicylsäure, Benzoesäure, Ameisensäure, Propionsäure, Pivalinsäure, Diethylessigsäure, Malonsäure, Bernsteinsäure, Pimelinsäure, Fumarsäure, Maleinsäure, Äpfelsäure, Sulfaminsäure, Phenylpropionsäure, Gluconsäure, Ascorbinsäure, Isonicotinsäure, Zitronensäure, Adipinsäure usw. Enthalten die Verbindungen der Formel I gleichzeitig saure und basische Gruppen im Molekül, so gehören neben den geschilderten Salzformen auch innere Salze oder Betaine (Zwitterionen) zu der Erfindung. Salze können aus den Verbindungen der Formel I nach üblichen, dem Fachmann bekannten Verfahren erhalten werden, beispielsweise durch Vereinigung mit einer organischen oder anorganischen Säure oder Base in einem Lösungsmittel oder Dispergiermittel, oder auch durch Anionenaustausch oder Kationenaustausch aus anderen Salzen. Die vorliegende Erfindung umfaßt auch alle Salze der Verbindungen der Formel I, die sich wegen geringer physiologischer Verträglichkeit nicht direkt für eine Verwendung in Arzneimitteln eignen, aber zum Beispiel als Zwischenprodukte für chemische Reaktionen oder für die Herstellung physiologisch verträglicher Salze in Betracht kommen. Unter physiologisch verträglichen Salzen von Verbindungen der Formel (I) werden beispielsweise deren organische als auch anorganische Salze, wie sie in Remington's Pharmaceutical Sciences (17. Auflage, Seite 1418 (1985)) beschrieben sind, verstanden. Aufgrund der physikalischen und chemischen Stabilität und der Löslichkeit sind für saure Gruppen unter anderem Natrium-, Kalium-, Calcium- und Ammoniumsalze bevorzugt; für basische Gruppen sind unter anderem Salze der Salzsäure, Schwefelsäure, Phosphorsäure oder von Carbonsäuren oder Sulfonsäuren, wie z.B. Essigsäure, Zitronensäure, Benzoesäure, Maleinsäure, Fumarsäure, Weinsäure und p-Toluolsulfonsäure bevorzugt.

Die vorliegende Erfindung umfaßt weiterhin alle Solvate von Verbindungen der Formel I, zum Beispiel Hydrate oder Addukte mit Alkoholen, sowie Derivate der Verbindungen der Formel I wie zum Beispiel Ester, und Pro-Drugs und aktive Metabolite.

Die Erfindung betrifft auch ein Verfahren zur Herstellung der neuen Verbindungen der Formel I, sowie deren physiologisch verträglichen Salze, das dadurch gekennzeichnet ist, daß man Verbindungen der Formel II 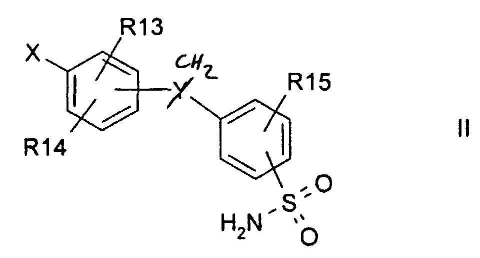 worin die Reste wie oben definiert sind, mit Bromcyan umsetzt. Die Reaktion erfolgt in einem dipolar aprotischen Lösungsmittel, das gegen Bromcyan stabil ist, zum Beispiel Acetonitril, DMA, TMU oder NMP mit einer starken Hilfsbase, die wenig nucleophil ist wie zum Beispiel K₂CO₃ oder Cs₂CO₃. Als Reaktionstemperatur kommt eine Temperatur zwischen 0 °C und dem Siedepunkt des verwendeten Lösungsmittels in Frage, bevorzugt ist eine Temperatur zwischen 60 °C und 120 °C.

Sulfonamidderivate der Formel II mit X gleich 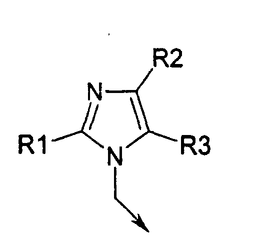 können hergestellt werden, indem Verbindungen der Formel III 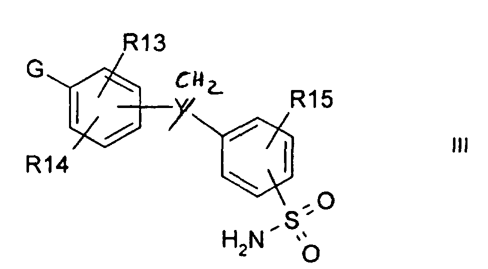 worin Y, R(13), R(14) und R(15) wie oben definiert sind und G beispielsweise CH₂Cl, CH₂Br, CH₂OH, CH₂OMs bedeutet und die Sulfonamidgruppe vorteilhaft in geschützter Form, beispielsweise als Dimethylaminomethylen-Derivat vorliegt, in literaturbekannter Weise (nukleophile Substitution oder Mitsunobu-Reaktion, vgl. J. Med. Chem. 1995, 38, 2357) mit Verbindungen der Formel IV 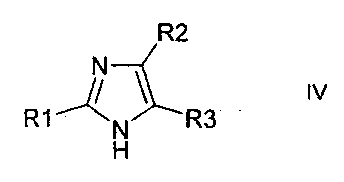 umgesetzt werden.

Verfahren zur Herstellung von Verbindungen der Formel IV sind u.a. bekannt aus EP-A 253310, EP-A 324377, US 5482957 und J. Med. Chem. 1995, 38, 2357-2377.

Verbindungen der Formel III können nach literaturbekannten Methoden hergestellt werden, indem man geeignete Arylderivate, die beispielsweise mit Methyl, Carbonsäureester, Formyl substituiert sind, aus denen nach dem Fachmann bekannten Verfahren die oben erwähnten Reste G erhalten werden können, beispielsweise in situ generierte Benzylzink-Derivate (vgl. J. Org. Chem. 1977, 42, 1821; J. Org. Chem. 1988, 53, 5789) mit substituierten Arylhalogeniden unter Pd(0)-Katalyse reagieren läßt.

Die erfindungsgemäßen Verbindungen der Formel I eignen sich als Inhibitoren des Natrium-abhängigen Bicarbonat/Chlorid-Austauschers (NCBE) bzw. des Natrium/Bicarbonat-Symporters.

In EP-A 855392 werden Imidazolderivate mit einer Biphenyl-sulfonylcyanamid-Seitenkette als NCBE-Inhibitoren beschrieben.

In EP-A 903339 und DE 19804251.5 werden substituierte Biphenylsulfonylcyanamidderivate als NCBE-Inhibitoren vorgeschlagen.

Den erfindungsgemäßen Verbindungen der Formel I ähnliche Verbindungen sind aus den US-Patentschriften 5,482,957 und 5,604,251 sowie EP-A 479479 bekannt, wo diese als blutdrucksenkende Angiotensin II-antagonisten beschrieben werden. Sie weisen jedoch nicht die nach der Erfindung stets vorhandene Sulfonylcyanamid-Seitenkette auf. Imidazolbiphenylderivate werden auch in WO9523792, WO9523791, EP-A 465368, EP-A 648763 beschrieben. Die bekannten Verbindungen sind Angiotensin II-Rezeptor-Antagonisten des Subtyps AT1, welche Wirkung bei den erfindungsgemäßen Verbindungen I nicht oder nur im geringen Ausmaß vorhanden ist.

Des weiteren betrifft die Erfindung die Verwendung einer Verbindung der Formel I zur Herstellung eines Medikaments zur Behandlung oder Prophylaxe von durch ischämische Zustände hervorgerufenen Krankheiten;
sowie die Verwendung einer Verbindung der Formel I zur Herstellung eines Medikaments zur Behandlung oder Prophylaxe des Herzinfarkts;
sowie die Verwendung einer Verbindung der Formel I zur Herstellung eines Medikaments zur Behandlung oder Prophylaxe der Angina Pectoris;
sowie die Verwendung einer Verbindung der Formel I zur Herstellung eines Medikaments zur Behandlung oder Prophylaxe von ischämischen Zuständen des Herzens;
sowie die Verwendung einer Verbindung der Formel I zur Herstellung eines Medikaments zur Behandlung oder Prophylaxe von ischämischen Zuständen des peripheren und zentralen Nervensystems und des Schlaganfalls;
sowie die Verwendung einer Verbindung der Formel I zur Herstellung eines Medikaments zur Behandlung oder Prophylaxe von ischämischen Zuständen peripherer Organe und Gliedmaßen;
sowie die Verwendung einer Verbindung der Formel I zur Herstellung eines Medikaments zur Behandlung von Schockzuständen;
sowie die Verwendung einer Verbindung der Formel I zur Herstellung eines Medikaments zum Einsatz bei chirurgischen Operationen und Organtransplantationen;
sowie die Verwendung einer Verbindung der Formel I zur Herstellung eines Medikaments zur Konservierung und Lagerung von Transplantaten für chirurgische Maßnahmen;
sowie die Verwendung einer Verbindung der Formel I zur Herstellung eines Medikaments zur Behandlung von Krankheiten, bei denen die Zellproliferation eine primäre oder sekundäre Ursache darstellt; und somit ihre Verwendung zur Herstellung eines Antiatherosklerotikums, eines Mittels gegen diabetische Spätkomplikationen, Krebserkrankungen, fibrotische Erkrankungen wie Lungenfibrose, Leberfibrose oder Nierenfibrose, Prostatahyperplasie;
sowie die Verwendung einer Verbindung der Formel I zur Herstellung eines Medikaments zur Behandlung von gestörtem Atemantrieb;
sowie ein Arzneimittel, gekennzeichnet durch einen wirksamen Gehalt einer Verbindung der Formel I.

Aufgrund der Inhibition des zellulären Na⁺-abhängigen Cl⁻/HCO₃⁻ - Austauschmechanismus und der damit potentiel verbundenn antiarrhythmischen Eigenschaften der erfindungsgemäßen Verbindungen der Formel I, die beispielsweise für die Behandlung von Krankheiten wichtig sind, welche bei Sauerstoffmangelerscheinungen auftreten eignen sich die Verbindungen der Formel I als antiarrhythmische Arzneimittel mit cardioprotektiver Komponente zur Infarktprophylaxe und der Infarktbehandlung sowie zur Behandlung der Angina Pectoris, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern können.

Wegen ihrer potentiell schützenden Wirkungen gegen pathologische hypoxische und ischämische Situationen können die erfindungsgemäßen Verbindungen der Formel I infolge Inhibition des zellulären Na⁺-abhängigen Cl⁻/HCO₃⁻-Austauschmechanismus bzw. des Natrium/Bicarbonat-Symporters als Arzneimittel zur Behandlung aller akuten oder chronischen durch Ischämie ausgelösten Schäden oder dadurch primär oder sekundär induzierten Krankheiten verwendet werden. Sie können akut oder chronisch sauerstoffmangelversorgte Organe durch Verringerung oder Verhinderung ischämisch induzierter Schäden schützen und eignen sich somit als Arzneimittel beispielsweise bei Thrombosen, Gefäßspasmen, Atherosklerose oder bei operativen Eingriffen (z.B. bei Organ-Transplantationen von Niere und Leber, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können) oder chronischem oder akutem Nierenversagen. Die Verbindungen der Formel I sind ebenfalls potentiell wertvolle, protektiv wirkende Arzneimittel bei der Durchführung angioplastischer operativer Eingriffe beispielsweise am Herzen wie auch an peripheren Gefäßen. Entsprechend ihrer potentiell protektiven Wirkung gegen ischämisch induzierte Schäden sind die Verbindungen auch als Arzneimittel zur Behandlung von Ischämien des Nervensystems, insbesondere des Zentralnervensystems, geeignet, wobei sie z. B. zur Behandlung des Schlaganfalls oder des Hirnödems verwendet werden können. Darüber hinaus eignen sich die erfindungsgemäßen Verbindungen der Formel I ebenfalls zur Behandlung von Formen des Schocks, wie beispielsweise des allergischen, cardiogenen, hypovolämischen und des bakteriellen Schocks.

Aufgrund einer potentiell stark inhibierenden Wirkung auf die Proliferationen von Zellen, beispielsweise der Fibroblasten- Zellproliferation und der Proliferation der glatten Gefäßmuskelzellen kommen die Verbindungen der Formel I als wertvolle Therapeutika für Krankheiten in frage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und -hyperplasien, insbesondere bei Prostatahyperplasie bzw. Prostatahypertrophie verwendet werden.

Es wurde gefunden, daß Inhibitoren des Na⁺-abhängigen Cl⁻/HCO₃⁻-Austauschers bzw. des Natrium/Bicarbonat-Symporters die Atmung durch eine Zunahme der Chemosensibiliät der Atmungs-Chemorezeptoren stimulieren können. Diese Chemorezeptoren sind in beträchtlichem Umfang für die Aufrechterhaltung einer geordneten Atemtätigkeit verantwortlich. Sie werden durch Hypoxie, pH-Abfall und Anstieg von CO₂ (Hyperkapnie) im Körper aktiviert und führen zu einer Anpassung des Atemminutenvolumens. Im Schlaf ist die Atmung besonders störanfällig und in hohem Maße abhängig von der Aktivität der Chemorezeptoren.

Eine Verbesserung des Atemantriebes durch Stimulation der Chemorezeptoren mit Substanzen, die den Na⁺-abhängigen Cl⁻/HCO₃⁻-Austausch hemmen, führt zu einer Verbesserung der Atmung bei folgenden klinischen Zuständen und Krankheiten: Gestörter zentraler Atemantrieb (z. B. zentrale Schlafapnoen, plötzlicher Kindstod, postoperative Hypoxie), muskulär-bedingte Atemstörungen, Atemstörungen nach Langzeitbeatmung, Atemstörungen bei Adaptation im Hochgebirge, obstruktive und gemischte Form der Schlafapnoen, akute und chronische Lungenkrankheiten mit Hypoxie und Hyperkapnie.

Arzneimittel, die eine Verbindung der Formel I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen der Formel I können dabei allein oder zusammen mit galenischen Hilfsstoffen zur Anwendung kommen, und zwar sowohl in der Veterinärals auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnem, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierungen für die Verabreichung in Form von Aerosolen oder Sprays sind geeignet z. B. Lösungen, Suspensionen oder Emulsionen des Wirkstoffes der Formel I in einem pharmazeutisch unbedenklichen Lösungsmittels, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew. %.

Die Dosierung des zu verabreichenden Wirkstoffs der Formel I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung der Formel I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Die Verbindungen der Formel I können als einziger Wirkstoffe oder in Kombination mit anderen pharmakologisch wirksamen Verbindungen eingesetzt werden.

Die Verbindungen der Formel I und/oder ihre physiologisch verträglichen Salze können zur Erzielung einer vorteilhaften therapeutischen Wirkung auch zusammen mit anderen pharmakologisch wirksamen Verbindungen zur Behandlung oder Prophylaxe der obengenannten Krankheitsbilder, insbesonders zur Behandlung von Herz-Kreislauf-Erkrankungen eingesetzt werden. Bevorzugt ist die Kombination mit Inhibitoren des Natrium/Wasserstoff-Austauschers (NHE) und/oder mit aktiven Substanzen aus anderen Herz-Kreislauf-Wirkstoffklassen.

Die Erfindung betrifft des weiteren die Kombination von a) NCBE-Inhibitoren der Formel I und/oder deren physiologisch verträglichen Salze mit NHE-Inhibitoren und/oder deren physiologisch verträglichen Salze; b) NCBE-Inhibitoren der Formel I und/oder deren physiologisch verträglichen Salze mit aktiven Substanzen aus anderen Herz-Kreislauf-Wirkstoffklassen und/oder deren physiologisch verträglichen Salze sowie c) von NCBE-Inhibitoren der Formel I und/oder deren physiologisch verträglichen Salze mit NHE-Inhibitoren und/oder deren physiologisch verträglichen Salze und mit aktiven Substanzen aus anderen Herz-Kreislauf-Wirkstoffklassen und/oder deren physiologisch verträglichen Salze.

Bei den bekannten und als NHE-Inhibitoren identifizierten Wirkstoffen handelt es sich um Guanidin-Derivate, vorzugsweise um Acylguanidine, unter anderem wie sie in Edward J. Cragoe, Jr., "DIURETICS, Chemistry, Pharmacology and Medicine", J. WILEY & Sons (1983), 303 - 341 beschrieben sind oder um die in EP 98115754.8 aufgeführten NHE-Inhibitoren.

Geeignete NHE-Inhibitoren sind beispielsweise auch Benzoylguanidine, wie sie in US 5292755, US 5373024, US 5364868, US 5591754, US 5516805, US 5559153,US 5571842, US 5641792, US 5631293, EP-A 577024, EP-A 602522, EP-A 602523, EP-A 603650, EP-A 604852, EP-A 612723, EP-A 627413, EP-A 628543, EP-A 640593, EP-A 640588, EP-A702001, EP-A 713864, EP-A 723956, EP-A 754680, EP-A 765868, EP-A 774459, EP-A 794171, EP-A 814077, EP-A 869116 beschrieben sind; ortho-substituierte Benzoylguanidine, wie sie in EP-A 556673, EP-A 791577, EP-A 794172 beschrieben sind; ortho-amino-substituierte Benzoylguanidine, wie sie in EP-A 690048 beschrieben sind; Isochinoline, wie sie in EP-A 590455 beschrieben sind; Benzokondensierte 5-Ring-Heterocyclen, wie sie in EP-A 639573 beschrieben sind; Diacylsubstituierte Guanidine, wie sie in EP-A 640587 beschrieben sind; Acylguanidine, wie sie in US 5547953 beschrieben sind; Perfluoralkylgruppen tragende Phenylsubstituierte Alkyl- bzw. Alkenyl-carbonsäure-Guanidine, wie sie in US 5567734, EP-A 688766 beschrieben sind; Heteroaroyl-guanidine, wie sie in EP-A 676395 beschrieben sind; Bizyklische Heteroaroyl-guanidine, wie sie in EP-A 682017 beschrieben sind; Indenoylguanidine, wie sie in EP-A 738712 beschrieben sind; Benzyloxycarbonyl-guanidine, wie sie in EP-A 748795 beschrieben sind; Fluorphenylgruppen tragende Phenylsubstituierte Alkenylcarbonsäure-Guanidine, wie sie in EP-A 744397 beschrieben sind; substituierte Zimtsäureguanidine, wie sie in EP-A 755919 beschrieben sind; Sulfonimidamide, wie sie in EP-A 771788 beschrieben sind; Benzoldicarbonsäurediguanidine, wie sie in EP-A 774458, EP-A 774457 beschrieben sind; Diarylcarbonsäure-diguanidine, wie sie in EP-A 787717 beschrieben sind; substituierte Thiophenylalkenylcarbonsäure-guanidine, wie sie in EP-A 790245 beschrieben sind; Bis-ortho-substituierte Benzoylguanidine, wie sie in EP-A 810207 beschrieben sind; Substituierte 1- oder 2-Naphthylguanidine, wie sie in EP-A 810205 und EP-A 810206 beschrieben sind; Indanylidinacetylguanidine, wie sie in EP-A 837055 beschrieben sind; Phenylsubstituierte Alkenylcarbonsäure-guanidine, wie sie in EP-A 825178 beschrieben sind; Aminopiperidyl-Benzoylguanidine, wie sie in EP-A 667341 beschrieben sind; Heterocycloxy-Benzylguanidine, wie sie in EP-A 694537 beschrieben sind; ortho-substituierte Benzoylguanidine, wie sie in EP704431 beschrieben sind; ortho-substituierte Alkyl-Benzylguanidine, wie sie in EP-A 699660 beschrieben sind; ortho-substituierte Heterocyclyl-Benzoylguanidine, wie sie in EP-A 699666 beschrieben sind; ortho-substituierte 5-Methylsulfonyl-benzoylguanidine, wie sie in EP-A 708088 beschrieben sind; ortho-substituierte 5-Alkylsulfonylbenzoylguanidine mit 4-amino Substituenten, wie sie in EP-A 723963 beschrieben sind; ortho-substituierte 5-Alkylsulfonyl-Benzoylguanidine mit 4-Mercapto Substituenten, wie sie in EP-A 743301 beschrieben sind; 4-Sulfonyl- oder 4-Sulphinyl-Benzylguanidine, wie sie in EP-A 758644 beschrieben sind; Alkenylbenzoylguanidine, wie sie in EP-A 760365 beschrieben sind; Benzoylguanidine mit annelierten, cyclischen Sulfonen, wie sie in DE 19548708 beschrieben sind; Benzoyl-, Polycyclische Aroyl- und Heteroaroyl-guanidine, wie sie in WO 9426709 beschrieben sind; 3-Aryl/Heteroaryl-Benzoylguanidine, wie sie in WO 9604241 beschrieben sind; 3-Phenyl-Benzoylguanidine mit einem basischen Amid in der 5-Position, wie sie in WO 9725310 beschrieben sind; 3-Dihalothienyloder 3-Dihalophenyl-Benzoylguanidine mit einem basichen Substituenten in der 5-Position, wie sie in WO 9727183 beschrieben sind; 3-Methylsulfonylbenzoylguanidinen mit bestimmten Aminosubstituenten in der 4-Position, wie sie in WO 9512584 beschrieben sind; Amilorid-Derivate, wie sie in WO 9512592 beschrieben sind; 3-Methylsulfonyl-benzoylguanidine mit bestimmten Aminosubstituenten in der 4-Position, wie sie in WO 9726253 beschrieben sind; Indoloylguanidine, wie sie in EP-A 622356 und EP-A 708091 beschrieben sind; Indoloylguanidine mit einem annelierten zusätzlichen Ringsystem, wie sie in EP 787728 beschrieben sind; Methylguanidin-Derivate, wie sie in WO 9504052 beschrieben sind; 1,4-Benzoxazinoylguanidine, wie sie in EP-A 719766 beschrieben sind; 5-Brom-2-Naphthoylguanidine, wie sie in JP 8225513 beschrieben sind; Quinolin-4-Carbonylguanidine mit einem Phenylrest in 2-Position, wie sie in EP-A 726254 beschrieben sind; Cinnamoylguanidine, wie sie in JP 09059245 beschrieben sind; Propenoylguanidine mit einem Naphthalin-Substituenten, wie sie JP 9067332 beschrieben sind; Propenoylguanidine mit Indolsubstituenten, wie sie JP 9067340 beschrieben sind; oder Heteroaryl-substituierte Acryloylguanidine, wie sie in WO 9711055 beschrieben sind, sowie deren physiologisch verträglichen Salze.

Bevorzugte NHE-Inhibitoren sind die in den genannten Publikationen als bevorzugt hervorgehobenen Verbindungen. Ganz besonders bevorzugt sind die Verbindungen Cariporid (HOE642), HOE 694, EMD 96785, FR 168888, FR 183998, SM-20550, KBR-9032, sowie deren physiologisch verträglichen Salze. Am meisten-bevorzugt ist Cariporid oder ein anderes physiologisch verträgliches Salz des N-(4-Isopropyl-3-methansulfonyl-benzoyl)-guanidin.

Beispiele für herzkreislaufaktive Wirkstoffklassen, die sich therapeutisch vorteilhaft mit NCBE-Inhibitoren kombinieren lassen oder zusätzlich mit Kombinationen von NCBE-Inhibitoren und NHE-Inhibitoren kombiniert werden können, sind Beta-Rezeptoren-Blocker, Calcium-Antagonisten, Angiotensin-Conversions-Enzym-Hemmer, Angiotensin-Rezeptorblocker, Schleifendiuretika, Thiaziddiuretika, kaliumsparende Diuretika, Aldosteronantagonisten, wie sie beispielsweise in der Blutdrucksenkung eingesetzt werden, sowie Herzglykoside oder andere kontraktionskraftverstärkende Mittel in der Behandlung der Herzinsuffizienz und des Congestive Heart Failures, sowie Antiarrhythmika der Klassen I - IV, Nitrate, K_{ATP}-Öffner, K_{ATP}-Blocker, Hemmer des Veratridin-aktivierbaren Natriumkanals usw. So sind zum Beispiel geeignet: die Betablocker Propanolol, Atenolol, Metoprolol; die Calciumantagonisten Diltiazem-Hydrochlorid, Verapamil-Hydrochlorid, Nifedipin; die ACE-Hemmer Captopril, Enalapril, Ramipril; Trandolapril, Quinapril, Spirapril, bevorzugt Ramipril oder Trandolapril; die Angiotensin II-Rezeptorantagonisten Losartan, Valsartan, Telmisartan, Eprosartan, Tasosartan, Candesartan, Irbesartan; die Schleifendiruetika Furosemid, Piretanid, Torasemid; die Thiazid-Diuretika Hydrochlorothiazid, Metolazon, Indapamid; die kaliumsparenden Diuretika Amilorid, Triamteren, Spironolacton; die Herzglykoside Digoxin, Digitoxin, Strophanthin; die Antiarrhythmika Amiodaron, Sotalol, Bretylium, Flecainid; das Nitrat Glyceroltrinitrat; die K⁺(ATP)-Öffner Cromakalim, Lemakalim, Nocorandil, Pinacidil, Minoxidil; die Hemmer des veratridin-aktivierbaren Na⁺-Kanals.

Ein Beispiel eines solchen besonders vorteilhaften Kombinationspartners mit NCBE-Inhibitoren der Formel I sind Blocker des nicht-inaktivierenden-Natriumkanals (Veratridin-aktivierbarer Natriumkanal). Die Kombinationen eines NCBE-Inhibitors der Formel I mit einem Blocker des nicht-inaktivierenden-Natriumkanals (Veratridin-aktivierbarer Natriumkanal) eignet sich zur Infarkt- und Re-infarktprophylaxe und der Infarktbehandlung sowie zur Behandlung der Angina Pectoris und der Inhibierung ischämisch induzierter Herzarrhythmien, der Tachykardie und der Entstehung und Aufrechterhaltung des Kammerflimmerns, wobei die Kombinationen eines NCBE-Inhibitors der Formel I mit einem Blocker des nicht-inaktivierenden-Natriumkanals auch präventiv die pathophysiologischen Vorgänge beim Entstehen ischämisch induzierter Schäden inhibieren oder stark vermindern. Wegen ihrer verstärkten schützenden Wirkungen gegen pathologische hypoxische und ischämische Situationen können die erfindungsgemäßen Kombinationen eines NCBE-Inhibitors der Formel I mit einem Blocker des nicht-inaktivierenden-Natriumkanals infolge verstärkter Inhibierung des Na⁺ Einstroms in die Zelle als Arzneimittel zur Behandlung aller akuten oder chronischen, durch Ischämie ausgelösten Schäden oder dadurch primär oder sekundär induzierter Krankheiten verwendet werden. Dies betrifft ihre Verwendung als Arzneimittel für operative Eingriffe, z. B. bei Organtransplantationen, wobei die Kombinationen eines NCBE-Inhibitors der Formel I mit einem Blocker des nicht-inaktivierenden-Natriumkanals sowohl für den Schutz der Organe im Spender vor und während der Entnahme, zum Schutz entnommener Organe beispielweise auch bei deren Lagerung in physiologischen Badflüssigkeiten, wie auch bei der Überführung in den Empfängerorganismus verwendet werden können. Die Kombinationen eines NCBE-Inhibitors der Formel I mit einem Blocker des nicht-inaktivierenden-Natriumkanals sind ebenfalls wertvolle, protektiv wirkende Arzneimittel bei der Durchführung angioplastischer operativer Eingriffe beispielweise am Herzen wie auch an peripheren Gefäßen. Entsprechend ihrer protektiven Wirkung gegen ischämisch induzierte Schäden sind die Kombinationen eines NCBE-Inhibitors der Formel I mit einem Blocker des nicht-inaktivierenden-Natriumkanals auch als Arzneimittel zur Behandlung von Ischämien des Nervensystems, insbesondere des Zentralnervensystems geeignet, wobei sie zur Behandlung des Schlaganfalls oder des Hirnödems geeignet sind. Darüber hinaus eignen sich die erfindungsgemäßen Kombinationen eines NCBE-Inhibitors der Formel I mit einem Blocker des nicht-inaktivierenden-Natriumkanals ebenfalls zur Behandlung von Formen des Schocks, wie beispielsweise des allergischen, cardiogenen, hypovolämischen und des bakteriellen Schocks.

Neben der Anwendung als fixe Kombination betrifft die Erfindung auch die gleichzeitige, getrennte oder zeitlich abgestufte Anwendung von NCBE-Inhibitoren der Formel I mit NHE-Inhibitoren und/oder einer zusätzlichen aktiven Substanzen aus einer anderen Herz-Kreislauf-Wirkstoffklasse zur Behandlung der obengenannten Krankheiten.

Die Erfindung betrifft desweiteren eine pharmazeutische Zubereitung, enthaltend a) einen NCBE-Inhibitor der Formel I und einen NHE-Inhibitor und/oder deren physiologisch verträglichen Salze; oder b) einen NCBE-Inhibitor der Formel I und zusätzlich eine aktive Substanz aus einer anderen Herz-Kreislauf-Wirkstoffklasse und/oder deren physiologisch verträglichen Salze; oder c) einen NCBE-Inhibitor der Formel I, einen NHE-Inhibitor und zusätzlich eine aktive Substanz aus einer anderen Herz-Kreislauf-Wirkstoffklasse, und/oder deren physiologisch verträglichen Salze.

Durch die kombinierte Anwendung kann der Effekt des einen Kombinationspartners durch den jeweiligen anderen Partner potenziert werden, d.h., die Wirkung und/oder Wirkdauer einer erfindungsgemäßen Kombination oder Zubereitung ist stärker bzw. anhaltender als die Wirkung und/oder Wirkdauer der jeweiligen Einzelkomponenten' (synergistischer Effekt). Dies führt bei einer kombinierten Anwendung zu einer Verringerung der Dosis der jeweiligen Kombinationspartner, verglichen mit der Einzelanwendung. Die erfindungsgemäßen Kombinationen und Zubereitungen besitzen demnach den Vorteil, daß die zu applizierenden Wirkstoffmengen signifikant reduziert und unerwünschte Nebenwirkungen eliminiert bzw. stark redzuziert werden können.

Die Erfindung betrifft ferner eine Handelspackung, enthaltend als pharmazeutischen Wirkstoff a) einen NCBE-Inhibitor der Formel I und einen NHE-Inhibitor und/oder deren physiologisch verträglichen Salze; oder b) einen NCBE-Inhibitor der Formel I und zusätzlich eine aktive Substanz aus einer anderen Herz-Kreislauf-Wirkstoffklasse und/oder deren physiologisch verträglichen Salze; oder c) einen NCBE-Inhibitor der Formel I, einen NHE-Inhibitor und zusätzlich eine aktive Substanz aus einer anderen Herz-Kreislauf Wrkstoffklasse und/oder deren physiologisch verträglichen Salze jeweils zusammen mit Instruktionen für die Verwendung dieser Wirkstoffe in Kombination zur gleichzeitigen, getrennten oder zeitlich abgestuften Anwendung in der Behandlung oder Prophylaxe der obengenannten Krankheitsbilder, insbesondere zur Behandlung von Herz-Kreislauf-Erkrankungen.

Die erfindungsgemäßen pharmazeutischen Zubereitungen können beispielsweise hergestellt werden, indem man entweder die Einzelkomponenten als Pulver intensiv mischt, oder indem man die Einzelkomponenten in den geeigneten Lösungsmittel wie beispielsweise einem niederen Alkohol auflöst und das Lösungsmittel anschließend entfernt.

Das Gewichstverhältnis vom NCBE-Inhibitor zum NHE-Inhibitor oder der herzkreislaufaktiven Substanz in den erfindungsgemäßen Kombinationen und Zubereitungen beträgt zweckmässigerweise 1:0,01 bis 1:100 , vorzugsweise 1:0,1 bis 1:10.

Die erfindungsgemäßen Kombination und Zubereitungen enthalten insgesamt vorzugsweise 0,5-99,5 Gew.-%, insbesondere 4-99 Gew.-% dieser Wirkstoffe.

Bei der erfindungsgemäßen Anwendung bei Säugern, vorzugsweise beim Menschen bewegen sich beispielsweise die Dosen der verschieden Wirkstoffkomponenten im Berreich von 0,001 bis 100 mg/kg/Tag.

### Liste der Abkürzungen:

- BCECF: 2',7'-Bis(2-carboxyethyl)-5,6-carboxyfluorescein
- Bn: Benzyl
- CH₂Cl₂: Dichlormethan
- DCI: Desorption-Chemical lonisation
- DIP: Diisopropylether
- DMA: Dimethylacetamid
- DME: Dimethoxyethan
- DMF: N,N-Dimethylformamid
- EE: Ethylacetat (EtOAc)
- El: electron impact
- eq: Äquivalent
- ES: Elektrospray-Ionisation
- Esneg: Elektrospray, negative Ionisation
- Et: Ethyl
- EtOH: Ethanol
- FAB: Fast Atom Bombardment
- Fmoc: 9-Fluorenylmethoxycarbonyl
- HEP: n-Heptan
- HOAc: Essigsäure
- Me: Methyl
- MeOH: Methanol
- mp: Schmelzpunkt
- MTB: Methyltertiärbutylether

- NCBE: Natriumabhängiger Chlorid/Bicarbonat-Austauscher
- NHE: Natrium/Wasserstoff-Austauscher
- NMP: N-Methylpyrrolidon
- RT: Raumtemperatur
- THF: Tetrahydrofuran
- TMU: N,N,N',N'-Tetramethylharnstoff
- Tol: Toluol
- ZNS: Zentralnervensystem

### Beispiele:

Allgemeine Vorschrift zur Herstellung von Sulfonylcyanamiden aus Sulfonamiden

Das Sulfonamid-Ausgangsmaterial wird in 10 ml / mmol wasserfreiem Acetonitril gelöst, 3 Mol-Äquivalente K₂CO₃ sowie ein Mol-Äquivalent einer 5 N Lösung von BrCN in Acetonitril zugetropft und bis zum vollständigen Umsatz unter Rückfluß erhitzt (typische Reaktionszeit 10 Minuten bis 6 Stunden). Das Reaktionsgemisch wird anschließend ohne weitere Aufarbeitung an Kieselgel chromatographiert.

### Beispiel 1:

### 2-Butyl-5-methylsulfanyl-3-[4-(3'-cyanaminosulfonyl-benzyl)-benzyl]-3H-imidazol-4-carbonsäure-ethylester

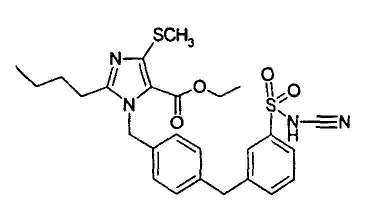

### Syntheseweg:

a) 3-Brom-N-dimethylaminomethylen-benzolsulfonamid durch Reaktion von 20.3 g 3-Brom-benzolsulfonamid mit 57 ml Dimethylformamid-dimethylacetal in 120 ml DMF bei RT während 3 Tagen. Nach wäßriger Aufarbeitung wird der weiße Niederschlag abfiltriert. Das Produkt wird im Vakuum bei 50 °C getrocknet und man erhält 20.5 g farblosen Feststoff, Fp. 122 °C, MS (ES): 291 (M+H)⁺
b) 4-[3-(Dimethylaminomethylen-sulfamoyl)-benzyl]-benzoesäuremethylester durch Kreuzkupplung von 3,5 g 3-Brom-N-dimethylaminomethylen-benzolsulfonamid (1a) mit 3 eq des Zinkreagenzes von 4-Brommethyl-benzoesäuremethylester in Gegenwart von 0.1 eq Pd(II)acetat, 0.2 eq Triphenylphosphin und 0.12 eq Kupfer(Í)iodid in 200 ml DMF bei 80°C für 3 h. Nach wäßriger Aufarbeitung wird das Solvens im Vakuum entfernt und der Rückstand an Kieselgel chromatographiert: 4.1 g farbloser Feststoff, Fp. 36-37°C,
   MS (ES) : 361 (M+H)⁺
c) 4-(3-Sulfamoyl-benzyl)-benzylalkohol aus 3.0 g 1b) durch Reduktion mit 1.2 g Lithiumalanat in 140 ml THF bei 0°C für 0.5h und anschließendem Rückflussieren für 2h. Wäßrige Aufarbeitung liefert 1.47 g farblosen Feststoff, Fp. 113-115°C.
d) 4-[3-(Dimethylaminomethylen-sulfamoyl)-benzyl]-benzylalkohol aus 1.7 g 1c) analog Reaktionsdurchführung 1a), 1.98 g farblose Kristalle, Fp. 92°C,
   MS (ES) : 333 (M+H)⁺
e) 2-Butyl-5-methylsulfanyl-3-[4-(3-dimethylaminomethylen-sulfamoyl-benzyl)-benzyl]-3H-imidazol-4-carbonsäure-ethylester durch nukleophile Substitution von in situ dargestelltem 4-[3-(Dimethylaminomethylen-sulfamoyl)-benzyl]-benzylmethansulfonat [(aus 0.7g 1d)] durch Reaktion mit 1eq Methansulfonsäurechlorid in Gegenwart von 2eq Triethylamin in Methylenchlorid bei 0°C für 2h, anschließendem Rühren bei RT für 1 h und anschließende Evaporation des Solvenz) mit 2 eq 2-Butyl-5-methylsulfanyl-3H-imidazol-4-carbonsäure-ethylester (J. Med. Chem. 1995, 38, 2357) in Gegenwart von 6 eq Kaliumkarbonat in 18 ml DMF bei 60°C für 16h. Wäßrige Aufarbeitung und anschließende säulenchromatographische Reinigung liefert 0.31g farbloses Öl,
   MS (ES) : 557 (M+H)⁺
f) 2-Butyl-5-methylsulfanyl-3-[4-(3-sulfamoyl-benzyl)-benzyl]-3H-imidazol-4-carbonsäure-ethylester durch Hydrolyse von 0.3 g 1e) mit 1 ml halbkonz. Salzsäure in 1ml Eisessig am Rückfluß für 2h. Zugabe von Wasser und anschließende Trocknung ergibt 0.27 g eines farblosen Harzes, MS (ES) : 502 (M+H)⁺
g) 2-Butyl-5-methylsulfanyl-3-[4-(3'-cyanaminosulfonyl-benzyl)-benzyl]-3Himidazol-4-carbonsäure-ethylester durch Cyanierung von 0.17 g 1f) analog allg. Vorschrift liefert 0.1 g farblosen Feststoff (Harz), Fp. 62°C, MS (ES): 527 (M+H)⁺.

### Beispiel 2:

### 2-Butyl-5-methylsulfanyl-3-[3-(3'-cyanaminosulfonyl-benzyl)-benzyl]-3H-imidazol-4-carbonsäure-ethylester

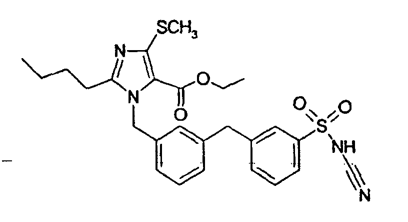

### Syntheseweg:

a) 3-[3-(Dimethylaminomethylen-sulfamoyl)-benzyl]-benzoesäuremethylester durch Kreuzkupplung von 3.1 g 1a) analog Durchführung 1b) ausgehend von 3eq 3-Brom-methyl-benzoesäuremethylester: 3.05 g farbloser Feststoff, Fp. 95°C,
   MS (ES) : 361 (M+H)⁺
b) 3-(3-Sulfamoyl-benzyl)-benzylalkohol aus 2.97 g 2a) durch Reduktion mit Lithium-alanat analog Durchführung 1c) liefert 1.9 g farblosen Feststoff, Fp. 94°C, MS (Cl) : 277 (M)⁺
c) 3-[3-(Dimethylaminomethylen-sulfamoyl)-benzyl]-benzylalkohol aus 1.39 g 2b) analog Reaktionsdurchführung 1a), 1.41 g farblose Kristalle, Fp. 99-100°C, MS (ES) : 333 (M+H)⁺
d) 2-Butyl-5-methylsulfanyl-3-[3-(3-dimethylaminomethylen-sulfamoyl-benzyl)-benzyl]-3H-imidazol-4-carbonsäure-ethylester durch nukleophile Substitution von in situ dargestelltem 3-[3-(Dimethylaminomethylen-sulfamoyl)-benzyl]-benzylmethansulfonat [(aus 0.28 g 2c)] analog Durchführung 1e), 0.15 g farbloses Harz,
   MS (ES) : 557 (M+H)⁺
e) 2-Butyl-5-methylsulfanyl-3-[3-(3-sulfamoyl-benzyl)-benzyl]-3H-imidazol-4-carbonsäure-ethylester durch Hydrolyse von 0.15 g 2d) analog Durchführung 1f) ergibt 0.13 g farblose Kristalle, Fp. 49°C, MS (ES) : 502 (M+H)⁺
f) 2-Butyl-5-methylsulfanyl-3-[3-(3'-cyanaminosulfonyl-benzyl)-benzyl]-3Himidazol-4-carbonsäure-ethylester durch Cyanierung von 0.12 g 2e) analog allg. Vorschrift liefert 0.07 g farblosen Feststoff (Harz), Fp. 172-174°C,MS (ES) : 527 (M+H)⁺

### Beispiel 3

### 2-Phenyl-4-formyl-5-chlor-3-[3-(3'-cyanaminosulfonyl-benzyl)-benzyl]-3H-imidazol

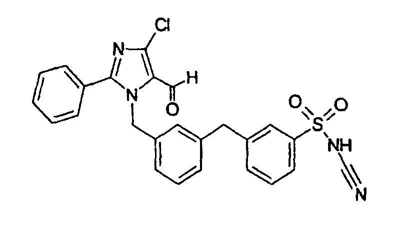

### Syntheseweg:

a) 2-Phenyl-4-formyl-5-chlor-3-[3-(3-dimethylaminomethylen-sulfamoyl-benzyl)-benzyl]-3H-imidazol durch nukleophile Substitution von in situ dargestelltem 3-[3-(Dimethylaminomethylen-sulfamoyl)-benzyl]-benzyl-methansulfonat [(aus 0.58 g 2c)] analog Durchführung 1e), jedoch unter Verwendung von 0.72 g 5-Chlor-4-formyl-2-phenyl-3H-imidazol (Chem. Pharm. Bull. 1976, 24(5), 960), 0.19 g farbloses Öl,
   MS (ES) : 521 (M+H)⁺
b) 2-Phenyl-4-formyl-5-chlor-3-[3-(3-sulfamoyl-benzyl)-benzyl]-3H-imidazol durch Hydrolyse von 0.17 g 2a) analog Durchführung 1f) ergibt 0.13 g farblose Kristalle, Fp. 83-85°C, MS (ES) : 466 (M+H)⁺
c) 2-Phenyl-4-formyl-5-chlor-3-[3-(3'-cyanaminosulfonyl-benzyl)-benzyl]-3Himidazol durch Cyanierung von 0.13 g 3b) analog allg. Vorschrift liefert 0.08 g farblosen Feststoff, Fp. 75°C, MS (ES): 491(M+H)⁺

### Beispiel 4:

### 2-[4-(4-Chlor-5-formyl-2-phenyl-imidazol-1-ylmethyl)-benzolsulfonyl]-benzolsulfonsäurecyanamid

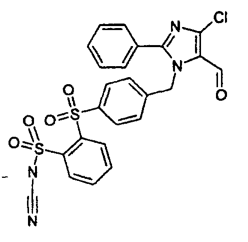

a) 2-p-Tolylsulfanyl-benzolsulfonamid
   3.0 g 2-Chlor-benzolsulfonamid, 2.0 g Thiokresol und 6.5 g K₂CO₃ werden in 30 ml DMF 6 h bei 100°C und anschließend 13 h bei 120°C gerührt. Das Reaktionsgemisch wird auf 200 ml Wasser gegossen und mit 500 ml EE extrahiert. Die organische Phase wird anschließend mit 100 ml einer gesättigten wäßrigen Na₂CO₃-Lösung gewaschen. Über MgSO₄ wird getrocknet und das Solvens im Vakuum entfernt. Chromatographie an Kieselgel mit DIP liefert 1.4 g weißer Kristalle, mp 122-124°C.
   R_{f}(DIP) = 0.36 MS (ES) : 280 (M+H)⁺
b) N-Dimethylaminomethylen-2-p-tolylsulfanyl-benzolsulfonamid
   1.4 g 2-p-Tolylsulfanyl-benzolsulfonamid werden in 10 ml Dimethoxymethyl-dimethylamin 2 h bei RT gerührt. Das Reaktionsgemisch wird anschließend auf 500 ml Wasser gegossen, 2 h nachgerührt, das Produkt abgesaugt und im Vakuum getrocknet. Man erhält 1.6 g weißer Kristalle, mp 151°C.
   R_{f}(MTB) = 0.28 MS (DCI) : 335 (M+H)⁺
c) N-Dimethylaminomethylen-2-(toluol-4-sulfonyl)-benzolsulfonamid
   1.5 g N-Dimethylaminomethylen-2-p-tolylsulfanyl-benzolsulfonamid werden in 50 ml CH₂Cl₂ gelöst und bei 0°C mit 2.6 g m-Chlorperbenzoesäure versetzt. 3 h wird bei RT nachgerührt, anschließend mit 100 ml einer gesättigten wäßrigen Na₂SO₃-Lösung versetzt und weitere 5 Minuten bei RT gerührt. Mit 150 ml CH₂Cl₂ wird verdünnt und 2 mal mit je 50 ml einer gesättigten wäßrigen Na₂CO₃-Lösung gewaschen. Über MgSO₄ wird getrocknet und das Solvens im Vakuum entfernt. Man erhält 1.5 g weißer Kristalle, mp 178°C.
   R_{f}(EE) = 0.44 MS (ES) : 367 (M+H)⁺
d) 2-(4-Brommethyl-benzolsulfonyl)-N-dimethylaminomethylen-benzolsulfonamid
   1.5 g N-Dimethylaminomethylen-2-(toluol-4-sulfonyl)-benzolsulfonamid, 0.74 g NBS und 20 mg Benzoylperoxid werden in 15 ml Chlorbenzol 2 h unter Rückfluß gekocht. Man läßt das Reaktionsgemisch abkühlen, verdünnt mit 100 ml Toluol und wäscht je einmal mit 20 ml einer gesättigten wäßrigen Na₂SO₃-Lösung und zweimal mit 50 ml einer gesättigten wäßrigen Na₂CO₃-Lösung. Über MgSO₄ wird getrocknet und das Solvens im Vakuum entfernt. Man erhält 1.6 g eines amorphen Feststoffs, der ohne weitere Reinigung weiter umgesetzt wird.
   R_{f}(EE) = 0.44 MS (ES) : 445 (M+H)⁺
e) 2-[4-(4-Chlor-5-formyl-2-phenyl-imidazol-1-ylmethyl)-benzolsulfonyl]-N-dimethylaminomethylen-benzolsulfonamid
   1.6 g 2-(4-Brommethyl-benzolsulfonyl)-N-dimethylaminomethylen-benzolsulfonamid, 680 mg 5-Chlor-2-phenyl-3H-imidazol-4-carbaldehyd und 1.4 g K₂CO₃ werden in 15 ml wasserfreiem DMF 3 Tage bei RT gerührt. Das Reaktionsgemisch wird anschließend mit 200 ml EE verdünnt und dreimal mit je 50 ml einer gesättigten wäßrigen Na₂CO₃-Lösung gewaschen. Über MgSO₄ wird getrocknet und das
   Solvens im Vakuum entfernt. Chromatographie an Kieselgel mit MTB liefert 240 mg eines farblosen Öls.
   R_{f}(MTB) = 0.08 MS (ES): 571 (M+H)⁺
f) 2-[4-(4-Chlor-5-formyl-2-phenyl-imidazol-1-ylmethyl)-benzolsulfonyl]-benzolsulfonamid
   230 mg 2-[4-(4-Chlor-5-formyl-2-phenyl-imidazol-1-ylmethyl)-benzolsulfonyl]-N-dimethylaminomethylen-benzolsulfonamid werden in 3 ml EtOH sowie 3 ml einer gesättigten wäßrigen HCl-Lösung 2 h unter Rückfluß gekocht. Das Reaktionsgemisch läßt man abkühlen, gießt auf 10 ml Wasser und filtriert das Produkt ab. Chromatographie an Kieselgel mit MTB liefert 151 mg eines amorphen Feststoffs.
   R_{f}(MTB) = 0.41 MS (ES) : 516 (M+H)⁺
g) 2-[4-(4-Chlor-5-formyl-2-phenyl-imidazol-1-ylmethyl)-benzolsulfonyl]-benzolsulfonsäurecyanamid
   145 mg 2-[4-(4-Chlor-5-formyl-2-phenyl-imidazol-1-ylmethyl)-benzolsulfonyl]-benzolsulfonamid, 117 mg K₂CO₃ und 56 µl einer 5 M Lösung von BrCN in Acetonitril werden in 2 ml wasserfreiem Acetonitril 90 Minuten unter Rückfluß gekocht. Man läßt das Reaktionsgemisch abkühlen, chromatographiert mit EE/MeOH 10:1 und erhält 90 mg eines amorphen Feststoffs.
   R_{f}(EE/MeOH 10:1) = 0.27 MS (ES) : 541 (M+H)⁺

### Pharmakologische Daten:

### Inhibition des Na⁺- abhängigen Cl⁻/HCO₃- - Austauschers (NCBE) in humanen

### Endothelzellen

Humane Endothelzellen (ECV-304) wurden aus Kulturflaschen mit Hilfe von Trypsin/EDTA-Puffer (0,05/0,02% in Phoshatpuffer) abgelöst und nach Zentrifugation (100g, 5 min) in einer gepufferten Salzlösung (mmol/l: 115 NaCl, 20 NH₄Cl, 5 KCl, 1 CaCl₂, 1 MgSO₄, 20 N-(2-Hydroxyethyl)piperazin-N=-2-ethansulfonsäure (HEPES), 5 Glucose und 1 g/l Rinderserumalbumin; pH 7,4) aufgenommen. Diese Zellsuspension wurde mit 5 µM BCECF-acetoxymethylester für 20 min bei 37°C inkubiert. Anschließend wurden die Zellen gewaschen und in einer Natrium- und Bicarbonat-freien Pufferlösung (mmol/l: 5 HEPES, 133,8 Cholinchlorid, 4,7 KCl, 1,25 MgCl₂, 0,97 K₂HPO₄, 0,23 KH₂PO₄, 5 Glucose; pH 7,4) resuspendiert.
Für die nachfolgende Fluoreszenzmessung im FLIPR (Fluorescent Imaging Plate Reader) wurden pro well einer 96-well-Mikrotiterplatte 100 µl dieser Zellsuspension mit jeweils 20000 Zellen einpipettiert und diese Mikrotiterplatte zentrifugiert (100g, 5 min).
Im FLIPR wurden nun aus einer weiteren vorbereiteten Mikrotiterplatte jeweils 100 µl Pufferlösung entnommen und in jedes der 96 wells der Meßplatte einpipettiert. Dabei wurde für eine 100% Kontrolle, d.h. eine Erholung des intrazellulären pH (pHᵢ) über den NCBE, eine Bicarbonat-und Natrium-haltige Pufferlösung (mmol/l: 5 HEPES, 93,8 NaCl, 40 NaHCO₃, 4,7 KCl, 1,25 CaCl₂, 1,25 MgCl₂, 0,97 Na₂HPO₄, 0,23 NaH₂PO₄, 5 Glucose; pH 7,4) verwendet, die 50 µM HOE 642 enthielt. Für eine 0% Kontrolle, d.h. keinerlei pHᵢ-Erholung, wurde eine Bicarbonat-freie, Natrium-haltige Pufferlösung (mmol/l: 5 HEPES, 133,8 NaCl, 4,7 KCl, 1,25 CaCl₂, 1,25 MgCl₂, 0,97 Na₂HPO₄, 0,23 NaH₂PO₄, 5 Glucose; pH 7,4) eingesetzt, welcher ebenfalls 50 µM HOE 642 zugefügt waren. Die erfindungsgemäßen Verbindungen der Formel (I) wurden in verschiedenen Konzentrationen der Natrium-und Bicarbonat-haltigen Lösung zugesetzt.
Nach Zugabe der Pufferlösungen zu den in der Meßplatte befindlichen farbstoffbeladenen, angesäuerten Zellen wurde der Anstieg der Fluoreszenzintensität, welcher einem Anstieg des pHᵢ entsprach, in jedem well der Mikrotiterplatte bestimmt. Die Kinetiken wurden dabei über einen Zeitraum von 2 Minuten bei 35°C aufgenommen.
Die Zunahme der Fluoreszenzintensitäten für unterschiedliche Konzentrationen der erfindungsgemäßen Verbindungen wurden auf die beiden Kontrollen bezogen und daraus die Hemmwirkung der Substanzen ermittelt.

### Ergebnisse

### Restaktivität des NCBE bei einer Inhibitorkonzentration von 10 µM

| Beispiel | Restaktivität in % |
|---|---|
| 1 | 11.1 |
| 2 | 35.2 |
| 3 | 24.3 |

## Patentansprüche

1. Verbindung der Formel (I), 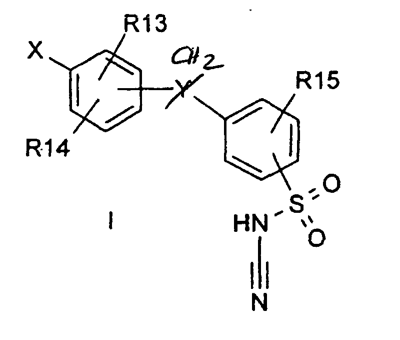 in welcher die Symbole folgende Bedeutung haben:
X 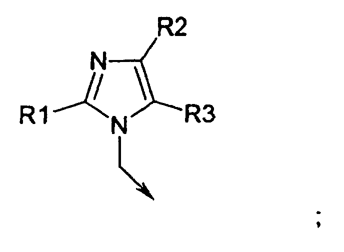
R(1) Alkyl mit 1, 2, 3 oder 4 C-Atomen oder Phenyl, das unsubstituiert oder
substituiert ist mit einem Rest aus der Reihe F, Cl, CF₃, Methyl oder Methoxy;
R(2) und R(3) unabhängig voneinander
Wasserstoff, F, Cl, CF₃, -CN, CO-R(24) oder O-R(25),
R(24) Wasserstoff, Alkyl mit 1,2,3 oder 4 C-Atomen, OR(26) oder
Phenyl, das unsubstituiert oder substituiert ist mit einem Rest aus der Reihe F, Cl, CF₃, Methyl oder Methoxy;
R(26) Wasserstoff, Methyl oder Ethyl;
R(25) Wasserstoff, Alkyl mit 1,2,3 oder 4 C-Atomen, Phenyl, das unsubstituiert oder substituiert ist mit einem Rest aus der Reihe F, Cl, CF₃, Methyl oder Methoxy;
oder
R(25) Heteroaryl, das unsubstituiert oder substituiert und mit einem Rest aus der Reihe F, Cl, CF₃, CH₃ oder Methoxy;
oder
R(2) und R(3) unabhängig voneinander
Alkyl mit 1, 2, 3 oder 4 C-Atomen, Cycloalkyl mit 3, 4, 5, 6 oder 7 C-Atomen;
oder
R(2) und R(3) unabhängig voneinander
Phenyl, das unsubstituiert oder substituiert ist mit einem Rest aus der Reihe F, Cl, CF₃, Methyl oder Methoxy;
oder
R(2) und R(3) unabhängig voneinander
Heteroaryl mit 1, 2, 3, 4, 5, 6, 7, 8 oder 9 C-Atomen, das unsubstituiert oder substituiert ist mit einem Rest aus der Reihe F, Cl, CF₃, CH₃ oder Methoxy;
oder
R(2) und R(3) unabhängig voneinander
-SO_{f}-R(37),
R(37) Alkyl mit 1,2,3 oder 4 C-Atomen oder Phenyl, das unsubstituiert oder substituiert ist mit einem Rest aus der Reihe F, Cl, CF₃, Methyl
oder Methoxy;
f Null oder 2;
R(13), R(14) und R(15) unabhängig voneinander
Wasserstoff, Methyl, F, Cl, CF₃, -CN, SO₂-R(79), CO-R(80) oder O-R(81);
R(79) und R(81) unabhängig voneinander
Alkyl mit 1, 2, 3 oder 4 C-Atomen oder Phenyl, das unsubstituiert ist oder substituiert ist mit einem Rest aus der Reihe F, Cl, CF₃, Methyl
oder Methoxy;
R(80) Wasserstoff, Methyl oder OR(84);
R(84) Wasserstoff oder Alkyl mit 1, 2, 3, oder 4 C-Atomen;
sowie deren physiologisch verträgliche Salze.

2. Verbindungen der Formel (I) gemäß Ansprüch 1 und/oder ihre physiologisch verträglichen Salze zur Anwendung als Arzneimittel.

3. Pharmazeutische Zubereitung, **gekennzeichnet durch** einen wirksamen Gehalt einer Verbindung der Formel (I) nach Anspruch 1 und/oder eines physiologisch verträglichen Salzes davon.

4. Pharmazeutische Zubereitung gemäß Anspruch 3, **dadurch gekennzeichnet, daß** sie zusätzlich eine wirksame Menge eines NHE-Inhibitors und/oder eine wirksame Substanz aus einer anderen Herz-Kreislauf-Wirkstoffklasse, und/oder deren physiologisch verträglichen Salze enthält.

5. Verbindungen der Formel I gemäß Anspruch 1 und/oder ihre physiologisch verträglichen Salze zur Verwendung als Inhibitor des Natrium-abhängigen Bicarbonat/Chlorid-Austauschers (NCBE).

6. Verbindungen der Formel I gemäß Anspruch 1 und/oder ihre physiologisch verträglichen Salze zur Verwendung in der Therapie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

7. Verbindungen der Formel I gemäß einem oder mehreren der Anspruch 1 und/oder ihre physiologisch verträglichen Salze zur Verwendung in der Therapie und/oder Prophylaxe von Krebs.

8. Pharmazeutische Zubereitung gemäß Anspruch 3 und/oder 4 zur Verwendung in der Therapie und/oder Prophylaxe des Herzinfarkts, der Angina Pectoris, von durch ischämische Zustände hervorgerufenen Krankheiten, von gestörtem Atemantrieb, von ischämischen Zuständen des Herzens, von ischämischen Zuständen des peripheren und zentralen Nervensystems und des Schlaganfalls, von ischämischen Zuständen peripherer Organe und Gliedmaßen, von Krankheiten, bei denen die Zellproliferation eine primäre oder sekundäre Ursache darstellt oder in der Behandlung von Schockzuständen, oder zum Einsatz bei chirurgischen Operationen und Organtransplantationen oder zur Konservierung und Lagerung von Transplantaten für chirurgische Maßnahmen.

9. Pharmazeutische Zubereitung gemäß Anspruch 3 und/oder 4 zur Verwendung in der Therapie und/oder Prophylaxe von Krebs.

## Claims

1. A compound of the formula (I) 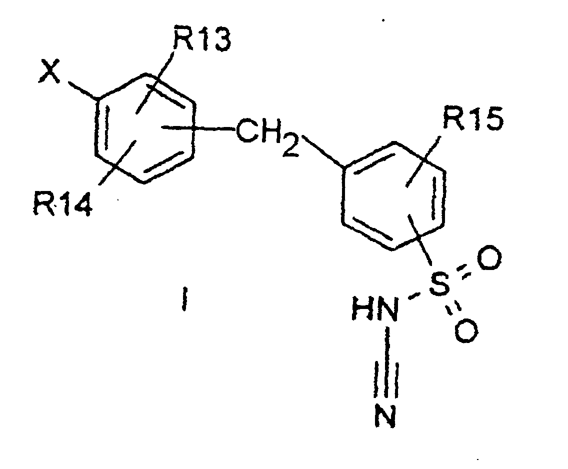 in which the symbols have the following meanings:
X is 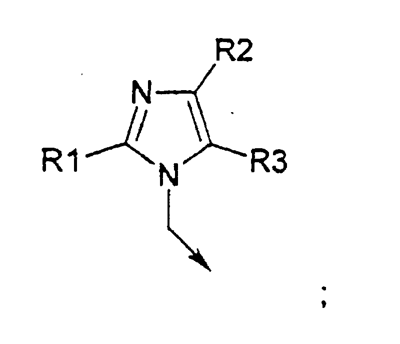
R(1) is alkyl having 1, 2, 3 or 4 carbon atoms or phenyl, which is unsubstituted or substituted by a radical from the series consisting of F, Cl, CF₃, methyl or methoxy;
R(2) and R(3) independently of one another are hydrogen, F, Cl, CF₃, -CN, CO-R(24) or O-R(25),
R(24) is hydrogen, alkyl having 1, 2, 3 or 4 carbon atoms, OR(26) or phenyl, which is unsubstituted or substituted by a radical from the series consisting of F, Cl, CF₃, methyl or methoxy;
R(26) is hydrogen, methyl or ethyl;
R(25) is hydrogen, alkyl having 1, 2, 3 or 4 carbon atoms, phenyl, which is unsubstituted or substituted by a radical from the series consisting of F, Cl, CF₃, methyl or methoxy;
or
R(25) is heteroaryl, which is unsubstituted or substituted by a radical from the series consisting of F, Cl, CF₃, CH₃ or methoxy;
or
R(2) and R(3) independently of one another are alkyl having 1, 2, 3 or 4 carbon atoms, cycloalkyl having 3, 4, 5, 6 or 7 carbon atoms;
or
R(2) and R(3) independently of one another are phenyl, which is unsubstituted or substituted by a radical from the series consisting of F, Cl, CF₃, methyl or methoxy;
or
R(2) and R(3) independently of one another are heteroaryl having 1, 2, 3, 4, 5, 6, 7, 8 or 9 carbon atoms, which is unsubstituted or substituted by a radical from the series consisting of F, Cl, CF₃, CH₃ or methoxy;
or
R(2) and R(3) independently of one another are
-SO_{f}-R(37),
R(37) is alkyl having 1, 2, 3 or 4 carbon atoms or phenyl, which is unsubstituted or substituted by a radical from the series consisting of F, Cl, CF₃, methyl or methoxy;
f is zero or 2;
R(13), R(14) and R(15) independently of one another are hydrogen, methyl, F, Cl, CF₃, -CN, SO₂-R(79), CO-R(80) or O-R(81);
R(79) and R(81) independently of one another are alkyl having 1, 2, 3 or 4 carbon atoms or phenyl, which is unsubstituted or substituted by a radical from the series consisting of F, Cl, CF₃, methyl or methoxy;
R(80) is hydrogen, methyl or OR(84);
R(84) is hydrogen or alkyl having 1, 2, 3 or 4 carbon atoms;
and their physiologically tolerable salts.

2. A compound of the formula (I) as claimed in claim 1 and/or its physiologically tolerable salts for use as a pharmaceutical.

3. A pharmaceutical preparation comprising an efficacious amount of a compound of the formula (I) as claimed in claim 1 and/or a physiologically tolerable salt thereof.

4. A pharmaceutical preparation as claimed in claim 3, which additionally contains an efficacious amount of an NHE inhibitor and/or an efficacious substance from another cardiovascular class of active compound, and/or their physiologically tolerable salts.

5. A compound of the formula I as claimed in claim 1 and/or its physiologically tolerable salts for use as an inhibitor of the sodium-dependent bicarbonate/chloride exchanger (NCBE).

6. A compound of the formula I as claimed in claim 1 and/or its physiologically tolerable salts for use in the therapy and/or prophylaxis of cardiac infarct, of angina pectoris, of illnesses caused by ischemic conditions, of impaired respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

7. A compound of the formula I as claimed in claim 1 and/or its physiologically tolerable salts for use in the therapy and/or prophylaxis of cancer.

8. A pharmaceutical preparation as claimed in claim 3 and/or 4 for use in the therapy and/or prophylaxis of cardiac infarct, of angina pectoris, of illnesses caused by ischemic conditions, of impaired respiratory drive, of ischemic conditions of the heart, of ischemic conditions of the peripheral and central nervous system and of stroke, of ischemic conditions of peripheral organs and limbs, of illnesses in which cell proliferation is a primary or secondary cause or in the treatment of states of shock, or for use in surgical operations and organ transplantations or for the preservation and storage of transplants for surgical measures.

9. A pharmaceutical preparation as claimed in claim 3 and/or 4 for use in the therapy and/or prophylaxis of cancer.

## Revendications

1. Composé de formule (I), 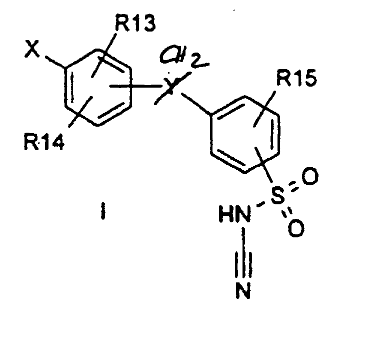 dans laquelle les symboles sont la signification suivante :
X représente 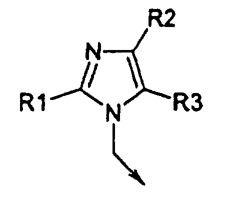
R(1) représente alkyle comprenant 1, 2, 3 ou 4 atomes de carbone ou phényle, qui est non substitué ou substitué par un radical de la série F, Cl, CF₃, méthyle ou méthoxy ;
R(2)et R(3) représentent, indépendamment l'un de l'autre
hydrogène, F, Cl, CF₃, -CN, CO-R(24) ou O-R(25),
R(24) représente hydrogène, alkyle comprenant 1,2,3 ou 4 atomes de carbone, OR(26) ou phényle, qui est non substitué ou substitué par un radical de la série F, Cl, CF₃, méthyle ou méthoxy ;
R(26) représente hydrogène, méthyle ou éthyle ;
R(25) représente hydrogène, alkyle comprenant 1,2,3 ou 4 atomes de carbone, phényle, qui est non substitué ou substitué par un radical de la série F, Cl, CF₃, méthyle ou méthoxy ;
ou
R(25) représente hétéroaryle, qui est non substitué ou substitué par un radical de la série F, Cl, CF₃, CH₃ ou méthoxy;
ou
R(2) et R(3) représentent, indépendamment l'un de l'autre
alkyle comprenant 1, 2, 3 ou 4 atomes de carbone, cycloalkyle comprenant 3, 4, 5, 6 ou 7 atomes de carbone ;
ou
R(2) et R(3) représentent, indépendamment l'un de l'autre,
phényle, qui est non substitué ou substitué par un radical de la série F, Cl, CF₃, méthyle ou méthoxy ;
ou
R(2) et R(3) représentent, indépendamment l'un de l'autre
hétéroaryle comprenant 1, 2, 3, 4, 5, 6, 7, 8 ou 9 atomes de carbone, qui est non substitué ou substitué par un radical de la série F, Cl, CF₃, CH₃ ou méthoxy ;
ou
R(2) et R(3) représentent, indépendamment l'un de l'autre,
-SO_{f}-R(37),
R(37) représente alkyle comprenant 1,2,3 ou 4 atomes de carbone ou phényle, qui est non substitué ou substitué par un radical de la série F, Cl, CF₃, méthyle ou méthoxy ;
f représente zéro ou 2 ;
R(13), R(14) et R(15) représentent indépendamment l'un de l'autre
hydrogène, méthyle, F, Cl, CF₃, -CN, SO₂-R(79), COR(80) ou O-R(81);
R(79) et R(81) représentent indépendamment l'un de l'autre alkyle comprenant 1, 2, 3 ou 4 atomes de carbone ou phényle, qui est non substitué ou substitué par un radical de la série F, Cl, CF₃, méthyle ou méthoxy ;
R(80) représente hydrogène, méthyle ou OR(84) ;
R(84) représente hydrogène ou alkyle comprenant 1, 2, 3 ou 4 atomes de carbone ;
ainsi que leurs sels physiologiquement acceptables.

2. Composés de formule (I) selon la revendication 1 et/ou leurs sels physiologiquement acceptables destinés à une utilisation comme médicament.

3. Préparation pharmaceutique, **caractérisée par** une teneur active en un composé de formule (I) selon la revendication 1 et/ou un sel physiologiquement acceptable de celui-ci.

4. Préparation pharmaceutique selon la revendication 3, **caractérisée en ce qu'**elle contient en outre une quantité active d'un inhibiteur de NHE et/ou une substance active d'une autre classe de substances actives cardio-vasculaires et/ou leurs sels physiologiquement acceptables.

5. Composés de formule I selon la revendication 1 et/ou leurs sels physiologiquement acceptables destinés à une utilisation comme inhibiteur de l'échangeur bicarbonate/chlorure dépendant du sodium (NCBE).

6. Composés dé formule I selon la revendication 1 et/ou leurs sels physiologiquement acceptables destinés à une utilisation dans la thérapie et/ou la prophylaxie de l'infarctus cardiaque, de l'angine de poitrine, de maladies provoquées par des états ischémiques, d'un trouble de la pompe respiratoire, d'états ischémiques du cerveau, d'états ischémiques du système nerveux périphérique et central et d'une attaque, d'états ischémiques d'organes périphériques et de membres, de maladies dans lesquelles la prolifération cellulaire représente une cause primaire ou secondaire ou dans le traitement d'états de choc
ou à une utilisation lors d'opérations chirurgicales et de transplantations d'organes ou à la conservation et l'entreposage de transplants pour des interventions chirurgicales.

7. Composés de formule I selon la revendication 1 et/ou leurs sels physiologiquement acceptables destinés à une utilisation dans la thérapie et/ou la prophylaxie du cancer.

8. Préparation pharmaceutique selon la revendication 3 et/ou 4 destinée à une utilisation dans la thérapie et/ou la prophylaxie de l'infarctus cardiaque, de l'angine de poitrine, de maladies provoquées par des états ischémiques, d'un trouble de la pompe respiratoire, d'états ischémiques du cerveau, d'états ischémiques du système nerveux périphérique et central et d'une attaque, d'états ischémiques d'organes périphériques et de membres, de maladies dans lesquelles la prolifération cellulaire représente une cause primaire ou secondaire ou dans le traitement d'états de choc ou à une utilisation lors d'opérations chirurgicales et de transplantations d'organes ou à la conservation et l'entreposage de transplants pour des interventions chirurgicales.

9. Préparation pharmaceutique selon la revendication 3 et/ou 4 destinée à une utilisation dans la thérapie et/ou la prophylaxie du cancer.
